(19) Europäisches Patentamt
European Patent Office
Office européen des brevets

(11) Publication number: 0 274 176 B1

(12) **EUROPEAN PATENT SPECIFICATION**

(45) Date of publication of patent specification: **27.05.92**

(51) Int. Cl.[5]: **A61K 9/18**, A61K 31/44

(21) Application number: **87300887.4**

(22) Date of filing: **02.02.87**

# (54) Sustained release capsule or tablet formulation.

(30) Priority: **09.01.87 IE 64/87**

(43) Date of publication of application:
**13.07.88 Bulletin 88/28**

(45) Publication of the grant of the patent:
**27.05.92 Bulletin 92/22**

(84) Designated Contracting States:
**BE CH DE ES FR GB IT LI NL SE**

(56) References cited:
**EP-A- 0 035 142**
**EP-A- 0 078 430**
**EP-A- 0 167 909**

(73) Proprietor: **ELAN CORPORATION P.L.C.**
**Monksland Industrial Estate**
**Athlone County Westmeath(IE)**

(72) Inventor: **Mulligan, Seamus**
**4 Beech Lawn, Monksland**
**Athlone, County Westmeath(IE)**
Inventor: **Sparks, Randall T.**
**2620 Pinebrook Drive**
**Gainsville, GA(US)**

(74) Representative: **Goldin, Douglas Michael et al**
**J.A. KEMP & CO. 14, South Square Gray's Inn**
**London WC1R 5EU(GB)**

EP 0 274 176 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a method for the manufacture of adsorbates for use in drug delivery systems and to the adsorbates and drug formulations thereby obtained.

It is frequently desirable to delay the release of an active substance from a pharmaceutical formulation in vivo. For example, it may be desirable to delay release of the active substance within the body so that the active substance is released at a particular target site. Various coated tablets are available which are resistant to gastric juices but which are readily soluble in the higher pH environment of the small intestine. Various controlled absorption pharmaceutical formulations are also available which have a particular dissolution pattern, resulting in a controlled absorption of the active substance and, therefore, more effective medication. For example, controlled absorption pellets of the latter type for oral administration are described in the Applicants' EP-A-O 122 077, EP-A-O 123 470, EP-A-O 156 077 and EP-A-O 149 920.

The use of many active substances in therapy is complicated by solubility problems and this is especially the case with the dihydropyridine group of drugs. Drugs of the latter type such as nifedipine are very poorly soluble in aqueous media and, whilst co-precipitates thereof with certain polymers are known to improve solubility, said co-precipitates normally require a polymer to active drug ratio exceeding 3:1 in order to be effective in producing products characterised by high bioavailability with prompt peak blood levels.

Pharmaceutical formulations based on an adsorbate of a drug within a cross-linked polymer, such as cross-povidone, are also known. Furthermore, solid, rapidly adsorbable medicament formulations comprising a dihydropyridine, polyvinylpyrrolidone with an average molecular weight of 15,000 to 50,000 and cross-linked insoluble polyvinylpyrrolidone are known from EP-A-O 167 909. Such formulations are rapidly absorbable and as such must be administered three or four times daily to achieve effective therapeutic levels of the active ingredient.

It is an object of the present invention to provide a sustained release capsule or tablet formulation for once daily administration wherein the bioavailability of an otherwise poorly bioavailable active substance is enhanced and effective controlled release formulations thereof can be produced.

Accordingly, the invention provides a sustained release capsule or tablet formulation suitable for once daily administration comprising an adsorbate of a mixture of 1 part by weight of a pharmaceutically useful dihydropyridine and from 0.1 to 10 parts by weight of a polyvinylpyrrolidone having an average molecular weight greater than 55,000 adsorbed on a cross-linked polyvinylpyrrolidone in a ratio of 1 part by weight of said mixture to 0.5 to 20 parts by weight of cross-linked polyvinylpyrrolidone, the adsorbate/cross-linked polyvinylpyrrolidone being blended with at least one polymer which gels in the presence of water, the amount of said polymer being effective to produce the desired sustained release effect.

The pharmaceutically useful dihydropyridines used in the present invention generally fall within the chemical formula

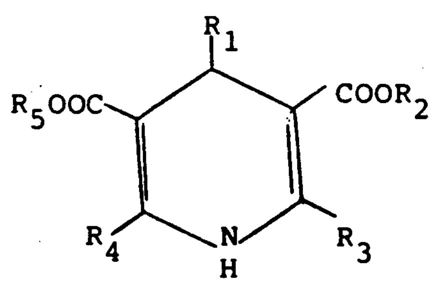

I

where $R_1$ is aryl or heteroaryl each of which may be substituted by various groups such as nitro and halogen whilst $R_2$, $R_3$, $R_4$ and $R_5$ which are the same or different, each represent alkyl groups which may be substituted by groups such as halogen, alkoxy, amino, alkylamino and arylalkylamino.

Preferred dihydropyridines covered by the general formula (I) are felodipine, nicardipine, nifedipine, nitrendipine, nimodipine, nisoldipine and 4-(2,1,3-benzoxadiazol-4-yl)-2,6-dimethyl-1,4-dihydro-3-isopropyloxycarbonyl-pyridine-5-carboxylic acid methyl ester.

The formation of the adsorbate may result in the dihydropyridine being in an amorphous state which can be verified by x-ray diffraction and, in addition, differential scanning calorimetery, which state is preferably retained in the sustained release formulations hereafter described.

The polyvinylpyrrolidone is preferably present in the adsorbate in an amount of 0.25 - 2 parts by weight relative to 1 part by weight of the dihydropyridine. Preferably the gel forming polymer is present in an

amount of 3 to 50% by weight of the formulation. Furthermore, the formulation preferably contains 1 part by weight of said mixture relative to 1 - 10 parts by weight of cross-linked polyvinylpyrrolidone.

The invention also provides a process for preparing a sustained release formulation as defined above, which comprises dissolving the dihydropyridine and the polyvinylpyrrolidone in a common solvent, mixing the solution thereby obtained with a given quantity of the cross-linked polyvinylpyrrolidone so as to permit adsorption of said dihydropyridine and said polyvinylpyrrolidone to said cross-linked polyvinylpyrrolidone, removing the solvent,and blending the resulting product with said polymer or mixture of polymers which gel in the presence of water and encapsulating or tabletting the resulting blend.

The solvent used is any pharmaceutically suitable co-solvent for the dihydropyridine and the polyvinylpyrrolidone.

The solvent is suitably selected from water, alcohols, ketones, halogenated aliphatic compounds, halogenated aromatic hydrocarbon compounds, aromatic hydrocarbon compounds and cyclic ethers or a mixture thereof.

Especially preferred solvents include water, hexane, heptane, methanol, ethanol, isopropyl alcohol, acetone, methylethyl ketone, methylisobutyl ketone, methylene chloride, chloroform, carbon tetrachloride, toluene, xylene and tetrahydrofuran.

The polyvinylpyrrolidone is chosen to modify the dissolution of the dihydropyridine from the cross-linked polyvinylpyrrolidone and also serves to prevent any crystallisation of the dihydropyridine in the polymer matrix structure of the adsorbate during dissolution. Principally, the polyvinylpyrrolidone serves to aid the sustained release mechanism of the capsule or tablet dosage form according to the invention.

The polyvinylpyrrolidone preferably has an average molecular weight in the range of 55,000 to 75,000. It is found that polyvinylpyrrolidones having a molecular weight greater than 55,000 have a viscosity which serves to sustain the release of active ingredient from the capsule or tablet formulation following administration. The greater the viscosity of the polyvinylpyrrolidone use, the slower the release of active ingredient.

An especially preferred cross-linked polyvinylpyrrolidone is cross-povidone (sold under the Trade Marks Polplasdone® XL (GAF) and Kollidon® CL (BASF)).

For forming capsules or tablets according to the invention, the adsorbate as defined above adsorbed on the cross-linked polyvinylpyrrolidone is granulated and blended with a polymer or mixture of polymers which gels in the presence of water, and optionally other ingredients. The blend thereby obtained can be tabletted or encapsulated according to conventional methods, thereby yielding a long acting matrix system which also exhibits improved drug absorption. Suitable polymers for blending with adsorbates for subsequent tabletting or encapsulation are inert polymers, which include both water soluble and water insoluble polymers such as, for example, polyvinyl alcohol, polyvinylpyrrolidone, hydroxyethylcellulose, hydroxypropylcellulose, hydroxypropylmethylcellulose, sodium carboxymethylcellulose, alkyl-celluloses such as methyl-and ethylcellulose, shellac, polymers sold under the Trade Mark Eudragit®, polyethylene glycol, sodium alginate, galactomannone or carboxypolymethylene or mixtures thereof.

Eudragit® polymers are polymeric lacquer substances based on acrylates and/or methacrylates. Especially suitable Eudragits® for use as the polymer which gels in water in the capsules or tablets according to the invention include co-polymers of acrylic and methacrylic acid esters which have varying permeability to water and active ingredient.

An especially suitable group of polymers is the polymers sold under the Trade Mark Methocel®.

If one wishes to delay release of the active ingredient <u>in vivo</u> in capsule or tablet form, a combination of water soluble and a water insoluble polymer or mixture of such polymers will be used, with the ratio of the water soluble to water insoluble polymer being varied to give the desired rate of release. Similarly, in the case of polymers/co-polymers of varying permeability, such as the Eudragits®, the permeability characteristic of the polymers/co-polymers will be chosen to give the desired rate of release.

The adsorbates according to the present invention result in improved drug delivery relative to known active drug adsorbates in a cross-linked polymer since the adsorbates in the formulations of the present invention yield a matrix system exhibiting both delayed or sustained release of active drug and improved absorption of said active drug <u>in vivo</u>.

The invention will be further illustrated with reference to the following Examples.

## EXAMPLE 1

Polyvinylpyrrolidone with an average molecular weight of 60,000 (0.75 kg) was dissolved in methylene chloride (8 kg). Nifedipine (1 kg) was then added to this solution and allowed to dissolve. The solution thereby obtained was then adsorbed onto cross-povidone (2.3 kg) and the solvent evaporated. The resulting powder was then passed through an oscillating granulator to obtain a finer particle size. X-ray diffraction and

differential scanning calorimetry studies were performed on the powder and demonstrated that the nifedipine was in an amorphous form. The powder 50% was then tabletted with the following ingredients.

| | |
|---|---|
| Methocel® K100LV (Trade Mark) | 8.0% |
| Avicel® pH101 (Trade Mark) | 41.5% |
| Magnesium stearate | 0.5% |

to obtain a tablet containing 20 mg of active ingredient. An x-ray diffraction pattern of the tablet was obtained which demonstrated the amorphous nature of the nifedipine had been retained.

In the above Example, the ratio of nifedipine, polyvinylpyrrolidone and cross-povidone may be altered within the limits which retain the amorphous nature of the drug. This also applies in the case of the subsequent Examples.

Furthermore, the Methocel® used may be Methocel® K4M, K15M, K100M, or E, J, F grades depending on the release characteristics desired.

The gel forming polymer may be used in amount of 3 - 50% with proportional changes in the precentage of adsorbate used. This also applies in the case of the subsequent Examples.

EXAMPLE 2

Polyvinylpyrrolidone with an average molecular weight of 60,000 (0.65 kg) was dissolved in isopropyl alcohol (10 kg). Nicardipine (1 kg) was then added to the solution and allowed to dissolve. The solution thereby obtained was then adsorbed onto cross-povidone (3 kg) and the solvent evaporated. The resulting powder was passed through an oscillating granulator to obtain a finer particle size. The powder (60%) was then tabletted with the following ingredients:

| | |
|---|---|
| Methocel® K100M (Trade Mark) | 8.0% |
| Avicel® pH101 (Trade Mark) | 31.5% |
| Magnesium stearate | 0.5% |

to obtain a tablet containing 60 mg of active ingredient.

EXAMPLE 3

The procedure of Example 1 was repeated except that the nifedipine was replaced by an equal amount (1 kg) of (4-(2,1,3-benzoxadiazol-4-yl)-2,6-dimethyl-1, 4-dihydro-3-isopropyloxycarbonyl-pyridine-5-carboxylic acid methyl ester (PN 200) and tablets containing 10 mg of active ingredient were produced.

EXAMPLE 4

Polyvinylpyrrolidone with an average molecular weight of 55,000 (0.75 kg) was dissolved in methylene chloride (12 kg) nifedipine (1 kg) was then added to this solution and allowed to dissolve. The solution thereby obtained was then adsorbed onto cross-povidone (3 kg) and the solvent evaporated. The resulting powder was then passed through an oscillating granulator to obtain a fine particle size. X-ray diffraction and differential scanning calorimetry studies showed that the drug was in amorphous form in this adsorbate. The powder (30%) was then tabletted with the following ingredients:

| | |
|---|---|
| Methocel® K100 LV (Trade Mark) | 10% |
| Avicel® pH101 (Trade Mark) | 59.5% |
| Magnesium stearate | 0.5% |

to obtain a tablet containing 20 mg active ingredient.

Similarly x-ray diffraction and differential scanning calorimetry studies show this product to be amorphous.

EXAMPLE 5

A nifedipine adsorbate was prepared as in the case of Example 4. The powder thus prepared (50%) was then blended with the following ingredients:

| | |
|---|---|
| Methocel® K100 M (Trade Mark) | 44.5% |
| Avicel® pH101 (Trade Mark) | 5.0% |
| Magnesium stearate | 0.5% |

and encapsulating to give capsules containing 20 mg of active ingredient.

EXAMPLE 6

Polyvinylpyrrolidone (1 kg) with an average molecular weight of 65,000 was dissolved in 10 kg methylene chloride. Felodipine (1.5 kg) was added and dissolved. The solution obtained was adsorbed onto cross-povidone (5 kg) and the solvent evaporated. The resulting powder was then passed through an oscillating granulator to obtain a fine particle size. X-ray diffraction and differential scanning calorimetry studies showed that the drug was in an amorphous form in this adsorbate. The powder (30%) was then tabletted with the following ingredients:

| | |
|---|---|
| Methocel® K15M (Trade Mark) | 30.0% |
| Avicel® pH101 (Trade Mark) | 39.5% |
| Magnesium stearate | 0.5% |

to obtain a tablet containing 10 mg active ingredient.

EXAMPLE 7

Example 1 was repeated except that the mixture adsorbed on the cross-povidone (50%) was tabletted with

| | |
|---|---|
| Sodium alginate | 15.0% |
| Pregelatinised starch | 33.5% |
| Talc | 1.5% |

to obtain tablets containing 50 mg of active ingredient.

EXAMPLE 8

Example 1 was repeated except that the mixture adsorbed on the cross-povidone contained nitrendipine and the final tablets contained 20 mg of the active ingredient.

EXAMPLE 9

Example 2 was repeated except that the mixture contained nimodipine and was tabletted with

| | |
|---|---|
| Lactose V.S.P. | 10.0% |
| Eudragit® R.S. | 10.0% |
| Eudragit® R.L. | 29.25% |
| Calcium stearate | 0.75% |

to form tablets containing 50 mg of nimodipine.

EXAMPLE 10

Example 1 was repeated except that the mixture adsorbed on the cross-povidone was tabletted with

| | |
|---|---|
| Dibasic calcium phosphate dihydrate N.F. | 15.0% |
| Ethylcellulose 100 c.p.s. | 15.0% |
| Polyethyleneglycol 6000 | 5.0% |
| Hydroxyethylcellulose | 29.0% |
| Calcium stearate | 1.0% |

## Claims

### Claims for the following Contracting States : BE, CH, DE, FR, GB, IT, LI, NL, SE

1. A sustained release capsule or tablet formulation suitable for once daily administration comprising an adsorbate of a mixture of 1 part by weight of a pharmaceutically useful dihydropyridine and from 0.1 to 10 parts by weight of a polyvinylpyrrolidone having an average molecular weight greater than 55,000 adsorbed on a cross-linked polyvinylpyrrolidone in a ratio of 1 part by weight of said mixture to 0.5 to 20 parts by weight of cross-linked polyvinylpyrrolidone, the adsorbate/cross-linked polyvinylpyrrolidone being blended with at least one polymer which gels in the presence of water, the amount of said polymer being effective to produce the desired sustained release effect.

2. A formulation according to claim 1, characterised in that said gel forming polymer is present in an effective amount from 3 to 50% by weight of the formulation.

3. A formulation according to claim 1 or 2, characterised in that the dihydropyridine is of the general formula (I)

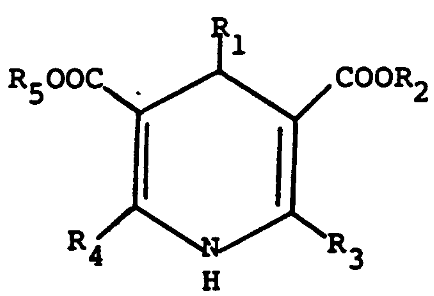

(I)

wherein $R_1$ is aryl or heteroaryl each of which is optionally substituted and $R_2$, $R_3$, $R_4$ and $R_5$, which may be the same or different, each represents an optionally substituted alkyl group.

4. A sustained release formulation according to claim 3, characterised in that in the dihydropyridine of the general formula I, $R_1$ is aryl or heteroaryl each of which is optionally substituted by a halogen atom or a nitro group and $R_2$, $R_3$, $R_4$ and $R_5$, which may be the same or different, each represents an alkyl group optionally substituted by a halogen atom or by an alkoxy, amino, alkylamino or aralkylamino group.

5. A sustained release formulation according to claim 3 or 4, characterised in that the dihydropyridine is selected from felodipine, nicardipine, nifedipine, nitrendipine, nimodipine, nisoldipine and 4-(2,1,3-benzoxadiazol-4-yl)-2,6-dimethyl-1,4-dihydro-3-isopropyloxycarbonyl- pyridine-5-carboxylic acid methyl ester.

6. A sustained release formulation according to any one of claims 1 to 5, characterised in that the polyvinylpyrrolidone has an average molecular weight in the range of 55,000 to 75,000 and is present in the adsorbate in an amount of 0.25 - 2 parts by weight relative to 1 part by weight of the dihydropyridine.

7. A sustained release formulation according to any one of claims 1 to 6, characterised in that it contains 1 part by weight of said mixture relative to 1-10 parts by weight of cross-linked polyvinylpyrrolidone.

8. A sustained release formulation according to any one of claims 1 to 7, characterised in that the polymer

which gels in the presence of water is selected from polyvinyl alcohol, polyvinylpyrrolidone, hydroxyethylcellulose, hydroxypropylcellulose, hydroxypropylmethylcellulose, sodium carboxymethylcellulose, alkylcelluloses, copolymers of acrylic and methacrylic acid esters, polyethylene glycol, sodium alginate, galactomannone and carboxypolymethylene and mixtures thereof.

**9.** A process for preparing a sustained release formulation according to any one of claims 1 - 8, characterised by dissolving the dihydropyridine and the polyvinylpyrrolidone in a common solvent, mixing the solution thereby obtained with the cross-linked polyvinylpyrrolidone so as to permit adsorption of said dihydropyridine and said polyvinylpyrrolidone to said cross-linked polyvinylpyrrolidone, removing the solvent, and blending the resulting product with said at least one polymer which gels in the presence of water and encapsulating or tabletting the resulting blend.

**10.** A process according to claim 9, wherein the solvent used is any pharmaceutically suitable co-solvent for the dihydropyridine and the polyvinylpyrrolidone and is especially selected from water, alcohols, ketones, halogenated aliphatic compounds, halogenated aromatic hydrocarbon compounds, aromatic hydrocarbon compounds and cyclic ethers or a mixture thereof.

**11.** A process according to claim 10, characterised in that the solvent is selected from water, hexane, heptane, methanol, ethanol, isopropyl alcohol, acetone, methylethyl ketone, methylisobutyl ketone, methylene chloride, chloroform, carbon tetrachloride, toluene, xylene and tetrahydrofuran.

**Claims for the following Contracting State : ES**

**1.** A process for preparing a sustained release capsule or tablet formulation suitable for once daily administration, comprising forming a mixture of 1 part by weight of a pharmaceutically useful dihydropyridine and from 0.1 to 10 parts by weight of a polyvinylpyrrolidone having an average molecular weight greater than 55,000, adsorbing said mixture on a cross-linked polyvinylpyrrolidone in a ratio of 1 part by weight of said mixture to 0.5 to 20 parts by weight of cross-linked polyvinylpyrrolidone, and blending the resultant adsorbate with a polymer or mixture of polymers which gel in the presence of water, the amount of said polymer or polymers being effective to produce the desired sustained release effect.

**2.** A process according to claim 1, characterised in that said gel forming polymer is included in an effective amount between 3 to 50% by weight of the formulation.

**3.** A process according to claim 1 or 2, characterised in that the dihydropyridine falls within the general formula (I)

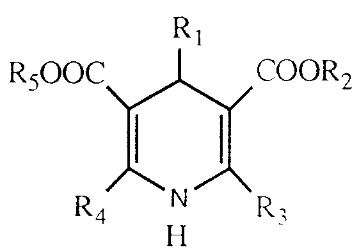

(I)

wherein $R_1$ is aryl or heteroaryl each of which is optionally substituted and $R_2$, $R_3$, $R_4$ and $R_5$, which may be the same or different, each represents an optionally substituted alkyl group.

**4.** A process according to claim 3, characterised in that in the dihydropyridine of the general formula I, $R_1$ is aryl or heteroaryl each of which is optionally substituted by a halogen atom or a nitro group and $R_2$, $R_3$, $R_4$ and $R_5$, which may be the same or different, each represents an alkyl group optionally substituted by a halogen atom or by an alkoxy, amino, alkylamino or aralkylamino group.

**5.** A process according to claim 3 or 4, characterised in that the dihydropyridine is selected from felodipine, nicardipine, nifedipine, nitrendipine, nimodipine, nisoldipine and 4-(2,1,3-benzoxadiazol-4-yl)-2,6-dimethyl-1,4-dihydro-3-isopropyloxycarbonyl-pyridine-5-carboxylic acid methyl ester.

6. A process according to any one of claims 1 to 5, characterised in that the polyvinylpyrrolidone, which has an average molecular weight in the range 55,000 to 75,000 is present in the adsorbate in an amount of 0.25 - 2 parts by weight relative to 1 part by weight of the dihydropyridine.

7. A process according to any one of claims 1 to 6, characterised in that it contains 1 part by weight of said mixture relative to 1-10 parts by weight of cross-linked polyvinylpyrrolidone.

8. A process according to any one of claims 1 to 7, characterised in that the polymer which gels in the presence of water is selected from polyvinyl alcohol, polyvinylpyrrolidone, hydroxyethylcellulose, hydroxypropylcellulose, hydroxypropylmethylcellulose, sodium carboxymethylcellulose, alkylcelluloses, copolymers of acrylic and methacrylic acid esters, polyethylene glycol, sodium alginate, galactomannone and carboxypolymethylene or mixtures thereof.

9. A process according to any preceding claim characterised by dissolving the dihydropyridine and the polyvinylpyrrolidone in a common solvent, mixing the solution thereby obtained with a given quantity of the cross-linked polyvinylpyrrolidone so as to permit adsorption of said dihydropyridine and said polyvinylpyrrolidone to said cross-linked polyvinylpyrrolidone, removing the solvent, and blending the resulting product with said polymer or mixture of polymers which gel in the presence of water and encapsulating or tabletting the resulting blend.

10. A process according to claim 9, wherein the solvent used is any pharmaceutically suitable co-solvent for the dihydropyridine and the polyvinylpyrrolidone and is especially selected from water, alcohols, ketones, halogenated aliphatic compounds, halogenated aromatic hydrocarbon compounds, aromatic hydrocarbon compounds and cyclic ethers or a mixture thereof.

11. A process according to claim 10, characterised in that the solvent is selected from water, hexane, heptane, methanol, ethanol, isopropyl alcohol, acetone, methylethyl ketone, methylisobutyl ketone, methylene chloride, chloroform, carbon tetrachloride, toluene, xylene and tetrahydrofuran.

**Revendications**
**Revendications pour les Etats contractants suivants : BE, CH, DE, FR, GB, IT, LI, NL, SE**

1. Formulation de capsule ou de comprimé à libération continue, adaptée pour une administration une fois par jour, comprenant un adsorbat d'un mélange constitué par 1 partie en poids d'une dihydropyridine pharmaceutiquement utilisable et 0,1 à 10 parties en poids de polyvinylpyrrolidone ayant une masse moléculaire moyenne supérieure à 55.000, adsorbé sur une polyvinylpyrrolidone réticulée dans un rapport de 1 partie en poids dudit mélange pour 0,5 à 20 parties en poids de la polyvinylpyrrolidone réticulée, l'adsorbat/polyvinylpyrrolidone réticulée étant mélangé avec au moins un polymère qui forme un gel en présence d'eau, la quantité dudit polymère étant efficace pour produire l'effet de libération continue souhaité.

2. Formulation selon la revendication 1, caractérisée en ce que ledit polymère formateur de gel est présent en une quantité active de 3 à 50 % en poids de la formulation.

3. Formulation selon la revendication 1 ou 2, caractérisée en ce que la dihydropyridine répond à la formule générale (I)

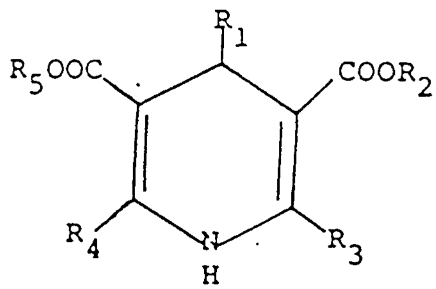

(I)

dans laquelle $R_1$ est un groupe aryle ou hétéroaryle qui peut être éventuellement substitué et $R_2$, $R_3$, $R_4$ et $R_5$, qui peuvent être identiques ou différents, représentent chacun un groupe alkyle éventuellement substitué.

**4.** Formulation à libération continue selon la revendication 3, caractérisée en ce que dans la dihydropyridine de formule générale I, $R_1$ représente un groupe aryle ou hétéroaryle qui est éventuellement substitué par un atome d'halogène ou un groupe nitro, et $R_2$, $R_3$, $R_4$ et $R_5$, qui peuvent être identiques ou différents, représentent chacun un groupe alkyle éventuellement substitué par un atome d'halogène ou par un groupe alcoxy, amino, alkylamino ou aralkylamino.

**5.** Formulation à libération continue selon la revendication 3 ou 4, caractérisée en ce que la dihydropyridine est choisie parmi la félodipine, la nicardipine, la nifédipine, la nitrendipine, la nimodipine, la nisoldipine et l'ester méthylique de l'acide 4-(2,1,3-benzoxadiazol-4-yl)-2,6-diméthyl-1,4-dihydro-3-isopropyloxycarbonyl-pyridine-5-carboxylique.

**6.** Formulation à libération continue selon l'une quelconque des revendications 1 à 5, caractérisée en ce que la polyvinylpyrrolidone présente une masse moléculaire moyenne dans la gamme de 55.000 à 75.000 et qu'elle est présente dans l'adsorbat en une proportion de 0,25 à 2 parties en poids pour 1 partie en poids de dihydropyridine.

**7.** Formulation à libération continue selon l'une quelconque des revendications 1 à 6, caractérisée en ce qu'elle contient une partie en poids dudit mélange pour 1 à 10 parties en poids de polyvinylpyrrolidone réticulée.

**8.** Formulation à libération continue selon l'une quelconque des revendications 1 à 7, caractérisée en ce que le polymère qui forme un gel en présence d'eau est choisi parmi l'alcool polyvinylique, la polyvinylpyrrolidone, l'hydroxyéthylcellulose, l'hydroxypropylcellulose, l'hydroxypropylméthylcellulose, la carboxyméthylcellulose de sodium, les alkylcelluloses, les copolymères des esters des acides acrylique et méthacrylique, le polyéthylèneglycol, l'alginate de sodium, la galactomannone et le carboxypolyméthylène ainsi que leurs mélanges.

**9.** Procédé pour la préparation d'une formulation à libération continue selon l'une quelconque des revendications 1 à 8, caractérisé par les étapes consistant à dissoudre la dihydropyridine et la polyvinylpyrrolidone dans un solvant commun, à mélanger la solution ainsi obtenue avec la polyvinylpyrrolidone réticulée de façon à permettre l'adsorption de ladite dihydropyridine et de ladite polyvinylpyrrolidone sur ladite polyvinylpyrrolidone réticulée, à éliminer le solvant et à mélanger le produit obtenu avec au moins un susdit polymère qui forme un gel en présence d'eau, et à encapsuler ou former en comprimé le mélange résultant.

**10.** Procédé selon la revendication 9, dans lequel le solvant utilisé est un solvant auxiliaire quelconque pharmaceutiquement acceptable de la dihydropyridine et de la polyvinylpyrrolidone et est en particulier choisi parmi l'eau, les alcools, les cétones, les composés aliphatiques halogénés, les composés hydrocarbures aromatiques halogénés, les composés hydrocarbures aromatiques et les éthers cycliques, ou un de leurs mélanges.

**11.** Procédé selon la revendication 10, caractérisé en ce que le solvant est choisi parmi l'eau, l'hexane,

l'heptane, le méthanol, l'éthanol, l'alcool isopropylique, l'acétone, la méthyléthylcétone, la méthylisobutylcétone, le chlorure de méthylène, le chloroforme, le tétrachlorure de carbone, le toluène, le xylène et le tétrahydrofurane.

**Revendications pour l'Etat contractant suivant : ES**

1. Procédé pour la préparation d'une formulation de capsule ou de comprimé à libération continue, appropriée pour une administration une fois par jour, comprenant la formation d'un mélange constitué par 1 partie en poids d'une dihydropyridine pharmaceutiquement utilisable et 0,1 à 10 parties en poids de polyvinylpyrrolidone ayant une masse moléculaire moyenne supérieure à 55.000, l'adsorbtion dudit mélange sur une polyvinylpyrrolidone réticulée dans un rapport de 1 partie en poids dudit mélange pour 0,5 à 20 parties en poids de la polyvinylpyrrolidone réticulée, et le mélange de l'adsorbat résultant avec un polymère ou un mélange de polymères qui forme un gel en présence d'eau, la quantité dudit polymère ou desdits polymères étant efficace pour produire l'effet de libération continue souhaité.

2. Procédé selon la revendication 1, caractérisé en ce que ledit polymère formateur de gel est inclus en une quantité active de 3 à 50 % en poids de la formulation.

3. Procédé selon la revendication 1 ou 2, caractérisé en ce que la dihydropyridine répond à la formule générale (I)

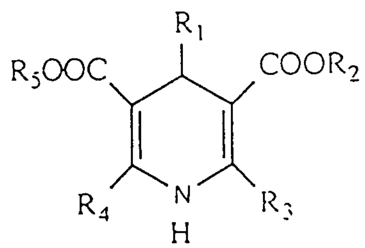

(I)

dans laquelle $R_1$ est un groupe aryle ou hétéroaryle qui peut être éventuellement substitué et $R_2$, $R_3$, $R_4$ et $R_5$, qui peuvent être identiques ou différents, représentent chacun un groupe alkyle éventuellement substitué.

4. Procédé selon la revendication 3, caractérisé en ce que dans la dihydropyridine de formule générale I, $R_1$ représente un groupe aryle ou hétéroaryle qui est éventuellement substitué par un atome d'halogène ou un groupe nitro, et $R_2$, $R_3$, $R_4$ et $R_5$, qui peuvent être identiques ou différents, représentent chacun un groupe alkyle éventuellement substitué par un atome d'halogène ou par un groupe alcoxy, amino, alkylamino ou aralkylamino.

5. Procédé selon la revendication 3 ou 4, caractérisé en ce que la dihydropyridine est choisie parmi la félodipine, la nicardipine, la nifédipine, la nitrendipine, la nimodipine, la nisoldipine et l'ester méthylique de l'acide 4-(2,1,3-benzoxadiazol-4-yl)-2,6-diméthyl-1,4-dihydro-3-isopropyloxycarbonyl-pyridine-5-carboxylique.

6. Procédé selon l'une quelconque des revendications 1 à 5, caractérisé en ce que la polyvinylpyrrolidone présente une masse moléculaire moyenne dans la gamme de 55.000 à 75.000 et qu'elle est présente dans l'adsorbat en une proportion de 0,25 à 2 parties en poids pour 1 partie en poids de dihydropyridine.

7. Procédé selon l'une quelconque des revendications 1 à 6, caractérisé en ce qu'elle contient une partie en poids dudit mélange pour 1 à 10 parties en poids de polyvinylpyrrolidone réticulée.

8. Procédé selon l'une quelconque des revendications 1 à 7, caractérisé en ce que le polymère qui forme un gel en présence d'eau est choisi parmi l'alcool polyvinylique, la polyvinylpyrrolidone, l'hydroxyéthylcellulose, l'hydroxypropylcellulose, l'hydroxypropylméthylcellulose, la carboxyméthylcellulose de sodium, les alkylcelluloses, les copolymères des esters des acides acrylique et méthacrylique, le

polyéthylèneglycol, l'alginate de sodium, la galactomannone et le carboxypolyméthylène ainsi que leurs mélanges.

9. Procédé selon l'une quelconque des revendications précédentes, caractérisé par les étapes consistant à dissoudre la dihydropyridine et la polyvinylpyrrolidone dans un solvant commun, à mélanger la solution ainsi obtenue avec la polyvinylpyrrolidone réticulée de façon à permettre l'adsorption de ladite dihydropyridine et de ladite polyvinylpyrrolidone sur ladite polyvinylpyrrolidone réticulée, à éliminer le solvant et à mélanger le produit obtenu avec ledit polymère ou mélange de polymères qui forme un gel en présence d'eau, et à encapsuler ou former en comprimé le mélange résultant.

10. Procédé selon la revendication 9, dans lequel le solvant utilisé est un solvant auxiliaire quelconque pharmaceutiquement acceptable de la dihydropyridine et de la polyvinylpyrrolidone et il est en particulier choisi parmi l'eau, les alcools, les cétones, les composés aliphatiques halogénés, les composés hydrocarbures aromatiques halogénés, les composés hydrocarbures aromatiques et les éthers cycliques, ou un de leurs mélanges.

11. Procédé selon la revendication 10, caractérisé en ce que le solvant est choisi parmi l'eau, l'hexane, l'heptane, le méthanol, l'éthanol, l'alcool isopropylique, l'acétone, la méthyléthylcétone, la méthylisobutylcétone, le chlorure de méthylène, le chloroforme, le tétrachlorure de carbone, le toluène, le xylène et le tétrahydrofurane.

**Patentansprüche**
**Patentansprüche für folgenden Vertragsstaaten : BE, CH, DE, FR, GB, IT, LI, NL, SE**

1. Formulierung für eine Kapsel oder Tablette mit verzögerter Freisetzung, geeignet für die einmal tägliche Verabreichung, welche ein Adsorbat einer Mischung aus 1 Gew.-Teil eines pharmazeutisch geeigneten Dihydropyridins und 0,1 bis 10 Gew.-Teilen eines Polyvinylpyrrolidons mit einem mittleren Molekulargewicht von mehr als 55 000, adsorbiert an einem vernetzten Polyvinylpyrrolidon in einem Verhältnis von 1 Gew.-Teil dieser Mischung zu 0,5 bis 20 Gew.-Teilen an vernetztem Polyvinylpyrrolidon, umfaßt, wobei das Adsorbat/vernetzte Polyvinylpyrrolidon mit wenigstens einem Polymer gemischt ist, das in Gegenwart von Wasser geliert, und wobei die Menge des Polymers ausreicht, den erwünschten Effekt der verzögerten Freisetzung zu erzeugen.

2. Formulierung nach Anspruch 1, dadurch gekennzeichnet, daß das gelbildende Polymer in einer effektiven Menge von 3 bis 50 Gew.-% der Formulierung vorhanden ist.

3. Formulierung nach Anspruch 1 oder 2, dadurch gekennzeichnet, daß das Dihydropyridin die allgemeine Formel (I) aufweist

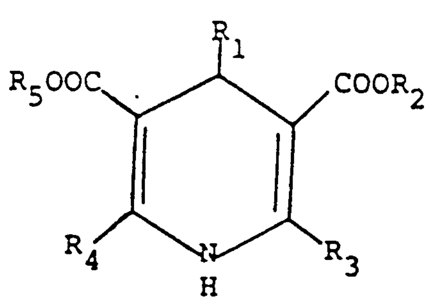

(I)

worin $R_1$ Aryl oder Heteroaryl ist, von denen jedes gegebenenfalls substituiert ist, und $R_2$, $R_3$, $R_4$ und $R_5$, die gleich oder verschieden sein können, jeweils eine gegebenenfalls substituierte Alkylgruppe darstellen.

4. Formulierung mit verzögerter Freisetzung nach Anspruch 3, dadurch gekennzeichnet, daß im Dihydropyridin der allgemeinen Formel I $R_1$ Aryl oder Heteroaryl ist, von denen jedes gegebenenfalls durch ein Halogenatom oder eine Nitrogruppe substituiert ist, und $R_2$, $R_3$, $R_4$ und $R_5$, die gleich oder verschieden

sein können, jeweils eine Alkylgruppe darstellen, die gegebenenfalls durch ein Halogenatom oder durch eine Alkoxy-, Amino-, Alkylamino- oder Aralkylaminogruppe substituiert sein können.

**5.** Formulierung mit verzögerter Freisetzung nach Anspruch 3 oder 4, dadurch gekennzeichnet, daß das Dihydropyridin aus Felodipin, Nicardipin, Nifedipin, Nitrendipin, Nimodipin, Nisoldipin und 4-(2,1,3-Benzoxadiazol-4-yl)-2,6-dimethyl-1,4-dihydro-3-isopropyloxycarbonyl-pyridin-5-carbonsäuremethylester ausgewählt ist.

**6.** Formulierung mit verzögerter Freisetzung nach einem der Ansprüche 1 bis 5, dadurch gekennzeichnet, daß das Polyvinylpyrrolidon ein mittleres Molekulargewicht im Bereich von 55 000 bis 75 000 hat und im Adsorbat in einer Menge von 0,25 bis 2 Gew.-Teilen, relativ zu 1 Gew.-Teil Dihydropyridin, vorhanden ist.

**7.** Formulierung mit verzögerter Freisetzung nach einem der Ansprüche 1 bis 6, dadurch gekennzeichnet, daß sie 1 Gew.-Teil der Mischung relativ zu 1 bis 10 Gew.-Teilen vernetztem Polyvinylpyrrolidon enthält.

**8.** Formulierung mit verzögerter Freisetzung nach einem der Ansprüche 1 bis 7, dadurch gekennzeichnet, daß das Polymer, das in Gegenwart von Wasser geliert, aus Polyvinylalkohol, Polyvinylpyrrolidon, Hydroxyethylcellulose, Hydroxypropylcellulose, Hydroxypropylmethylcellulose, Natriumcarboxymethylcellulose, Alkylcellulosen, Copolymeren von Acryl- und Methacrylsäureestern, Polyethylenglykol, Natriumalginat, Galactomannon und Carboxypolymethylen sowie Mischungen davon ausgewählt ist.

**9.** Verfahren zur Herstellung einer Formulierung mit verzögerter Freisetzung nach einem der Ansprüche 1 bis 8, gekennzeichnet durch das Auflösen des Dihydropyridins und des Polyvinylpyrrolidons in einem gewöhnlichen Lösungsmittel, Mischen der dadurch erhaltenen Lösung mit dem vernetzten Polyvinylpyrrolidon, um die Adsorption des Dihydropyridins und des Polyvinylpyrrolidons auf dem vernetzten Polyvinylpyrrolidon zu ermöglichen, Entfernen des Lösungsmittels und Mischen des resultierenden Produkts mit dem wenigstens einen Polymer, das in Gegenwart von Wasser geliert, sowie Einkapseln oder Tablettieren der resultierenden Mischung.

**10.** Verfahren nach Anspruch 9, worin das verwandte Lösungsmittel ein pharmazeutisch geeignetes Co-Lösungsmittel für das Dihydropyridin und das Polyvinylpyrrolidon ist und insbesondere aus Wasser, Alkoholen, Ketonen, halogenierten aliphatischen Verbindungen, halogenierten aromatischen Kohlenwasserstoffverbindungen, aromatischen Kohlenwasserstoffverbindungen und cyclischen Ethern oder Mischungen davon ausgewählt wird.

**11.** Verfahren nach Anspruch 10, dadurch gekennzeichnet, daß das Lösungsmittel aus Wasser, Hexan, Heptan, Methanol, Ethanol, Isopropylalkohol, Aceton, Methylethylketon, Methylisobutylketon, Methylenchlorid, Chloroform, Tetrachlorkohlenstoff, Toluol, Xylol und Tetrahydrofuran ausgewählt wird.

**Patentansprüche für folgenden Vertragsstaat : ES**

**1.** Verfahren zur Herstellung einer Formulierung für eine Kapsel oder Tablette mit verzögerter Freisetzung, geeignet für die einmal tägliche Verabreichung, durch Bilden einer Mischung aus 1 Gew.-Teil eines pharmazeutisch geeigneten Dihydropyridins und 0,1 bis 10 Gew.-Teilen eines Polyvinylpyrrolidons mit einem mittleren Molekulargewicht von mehr als 55 000, Adsorbieren dieser Mischung auf einem vernetzten Polyvinylpyrrolidon in einem Verhältnis von 1 Gew.-Teil der Mischung zu 0,5 bis 20 Gew.-Teilen vernetztem Polyvinylpyrrolidon sowie Mischen des resultierenden Adsorbats mit einem Polymer oder einer Mischung von Polymeren, die in Gegenwart von Wasser gelieren, wobei das Polymer oder die Polymere den erwünschten Effekt der verzögerten Freisetzung effektiv bewirken.

**2.** Verfahren nach Anspruch 1, dadurch gekennzeichnet, daß das gelbildende Polymer in einer effektiven Menge zwischen 3 und 50 Gew.-% der Formulierung einbezogen ist.

**3.** Verfahren nach Anspruch 1 oder 2, dadurch gekennzeichnet, daß das Dihydropyridin unter die allgemeine Formel (I) fällt

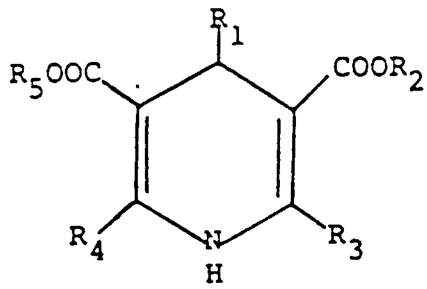

(I)

worin $R_1$ Aryl oder Heteroaryl ist, von denen jedes gegebenenfalls substituiert ist, und $R_2$, $R_3$, $R_4$ und $R_5$, die gleich oder verschieden sein können, jeweils eine gegebenenfalls substituierte Alkylgruppe darstellen.

4. Verfahren nach Anspruch 3, dadurch gekennzeichnet, daß im Dihydropyridin der allgemeinen Formel I $R_1$ Aryl oder Heteroaryl ist, von denen jedes gegebenenfalls durch ein Halogenatom oder eine Nitrogruppe substituiert ist, und $R_2$, $R_3$, $R_4$ und $R_5$, die gleich oder verschieden sein können, jeweils eine Alkylgruppe darstellen, die gegebenenfalls durch ein Halogenatom oder durch eine Alkoxy-, Amino-, Alkylamino- oder Aralkylaminogruppe substituiert sein kann.

5. Verfahren nach Anspruch 3 oder 4, dadurch gekennzeichnet, daß das Dihydropyridin aus Felodipin, Nicardipin, Nifedipin, Nitrendipin, Nimodipin, Nisoldipin und 4-(2,1,3-Benzoxadiazol-4-yl)-2,6-dimethyl-1,4-dihydro-3-isopropyloxycarbonylpyridin-5-carbonsäuremethylester ausgewählt ist.

6. Verfahren nach einem der Ansprüche 1 bis 5, dadurch gekennzeichnet, daß das Polyvinylpyrrolidon, das ein mittleres Molekulargewicht im Bereich von 55 000 bis 75 000 aufweist, im Adsorbat in einer Menge von 0,25 bis 2 Gew.-Teilen, relativ zu 1 Gew.-Teil Dihydropyridin, vorhanden ist.

7. Verfahren nach einem der Ansprüche 1 bis 6, gekennzeichnet durch 1 Gew.-Teil der Mischung relativ zu 1 bis 10 Gew.-Teilen an vernetztem Polyvinvlpyrrolidon.

8. Verfahren nach einem der Ansprüche 1 bis 7, dadurch gekennzeichnet, daß das Polymer, das in Gegenwart von Wasser geliert, aus Polyvinylalkohol, Polyvinylpyrrolidon, Hydroxyethylcellulose, Hydroxypropylcellulose, Hydroxypropylmethylcellulose, Natriumcarboxymethylcellulose, Alkylcellulosen, Copolymeren von Acryl- und Methacrylsäureestern, Polyethylenglykol, Natriumalginat, Galactomannon und Carboxypolymethylen oder Mischungen davon ausgewählt wird.

9. Verfahren nach einem der vorstehenden Ansprüche, gekennzeichnet durch das Auflösen des Dihydropyridins und des Polyvinylpyrrolidons in einem gewöhnlichen Lösungsmittel, Mischen der dadurch erhaltenen Lösung mit einer gegebenen Menge an vernetztem Polyvinylpyrrolidon, um die Adsorption des Dihydropyridins und des Polyvinylpyrrolidons auf dem vernetzten Polyvinylpyrrolidon zu ermöglichen, Entfernen des Lösungsmittels und Mischen des resultierenden Produkts mit dem Polymer oder der Mischung von Polymeren, die in Gegenwart von Wasser gelieren, sowie Einkapseln oder Tablettieren der resultierenden Mischung.

10. Verfahren nach Anspruch 9, worin das verwandte Lösungsmittel ein beliebiges pharmazeutisch geeignetes Co-Lösungsmittel für Dihydropyridin und Polyvinylpyrrolidon ist und insbesondere aus Wasser, Alkoholen, Ketonen, halogenierten aliphatischen Verbindungen, halogenierten aromatischen Kohlenwasserstoffverbindungen,aromatischen Kohlenwasserstoffverbindungen und cyclischen Ethern oder einer Mischung davon ausgewählt wird.

11. Verfahren nach Anspruch 10, dadurch gekennzeichnet, daß das Lösungsmittel aus Wasser, Hexan, Heptan, Methanol, Ethanol, Isopropylalkohol, Aceton, Methylethylketon, Methylisobutylketon, Methylenchlorid, Chloroform, Tetrachlorkohlenstoff, Toluol, Xylol und Tetrahydrofuran ausgewählt wird.